# EUROPÄISCHE PATENTSCHRIFT

(11) **EP 2 425 807 B1**
(45) Veröffentlichungstag und Bekanntmachung des Hinweises auf die Patenterteilung: **02.01.2013**
(21) Anmeldenummer: 10401161.4
(22) Anmeldetag: 07.09.2010
(51) Int. Cl.: A61G 9/02, A61L 2/18

(54) **Reinigungseinrichtung für Steckbecken**
Cleaning device for bedpan
Apparail de nettoyage pour des bassins de lit

(43) Veröffentlichungstag der Anmeldung: 07.03.2012
(73) Patentinhaber: Miele & Cie. KG, 33332 Gütersloh (DE)
(72) Erfinder: Dittert, Jörg, 33613 Bielefeld (DE); Horstmann, Peter, 33332 Gütersloh (DE); Ramrath, Frank, 33829 Borgholzhausen (DE); Schilling, Olaf, 49163 Bohmte (DE); Mracek, Maik, 33607 Bielefeld (DE); Scheele, Roland, 33613 Bielefeld (DE)

(56) Entgegenhaltungen:
- EP-A1- 0 738 545
- DE-A1- 3 628 793
- DE-B- 1 239 433
- US-A- 4 015 614

## Beschreibung

Die Erfindung betrifft eine Reinigungseinrichtung für Steckbecken, mit einem Gehäuse, das einen Reinigungsraum bereitstellt, wobei der Reinigungsraum eine Beschickungsöffnung aufweist, die mittels einer Tür fluiddicht verschließbar ist, welche Tür reinigungsraumseitig eine Steckbeckenaufnahme trägt.

Reinigungseinrichtungen für Steckbecken sind aus dem Stand der Technik an sich bekannt, etwa aus der DE3628793A1 und der DE1239433B. Sie verfügen über ein Gehäuse, das einen Reinigungsraum bereitstellt. Der Reinigungsraum ist über eine Beschickungsöffnung zugänglich, die mittels einer Tür fluiddicht verschließbar ist. Die Tür ist als Schwenktür ausgebildet und trägt reinigungsraumseitig eine Steckbeckenaufnahme.

Im bestimmungsgemäßen Verwendungsfall wird zur Beschickung der Reinigungseinrichtung zunächst die Tür in eine Offenstellung verschwenkt. Das zu reinigende Steckbecken wird dann in die von der Tür getragene Steckbeckenaufnahme eingeführt. Als dann ist die Tür in die Schließstellung zu verschwenken, infolge dessen das zu reinigende Steckbecken in den vom Gehäuse der Reinigungseinrichtung bereitgestellten Reinigungsraum einschwenkt. Nach einem fluiddichten Verschließen des Reinigungsraums mittels der Tür kann ein bestimmungsgemäßer Reinigungsvorgang durchgeführt werden. Nach erfolgter Reinigung kann die Tür wieder geöffnet und das Steckbecken aus der Steckbeckenaufnahme entfernt werden.

Aus dem Stand der Technik sind in ihrer geometrischen Ausgestaltung unterschiedliche Steckbecken bekannt geworden. Es gibt auch solche Steckbecken, die neben dem eigentlichen Steckbeckengrundkörper über einen Deckel verfügen. Ein solcher Deckel dient insbesondere dazu, den Inhalt eines Steckbeckens, beispielsweise Fäkalien, andere Körperflüssigkeiten, Toilettenpapierreste oder dergleichen abzudecken. Eine solche Deckel-Lösung vermindert insbesondere Geruchsbelästigungen und dient darüber hinaus dazu, den unter Umständen unansehnlichen Steckbeckeninhalt abzudecken, was für das handhabende Personal angenehmer ist.

Eine Reinigungsvorrichtung für Steckbecken mit einem Steckbeckengrundkörper und einem Deckel ist beispielsweise in der US4015614 beschrieben.

Bevor ein mit einem Deckel ausgerüstetes Steckbecken in eine Reinigungseinrichtung der vorbeschriebenen Art eingesetzt werden kann, ist der Deckel manuell zu entfernen und zusammen mit dem Steckbeckengrundkörper in die Steckbeckenaufnahme einzustecken.

Diese für eine ordnungsgemäße Bestückung der Steckbeckenaufnahme erforderliche Handhabung wird zumeist als unangenehm empfunden. Darüber hinaus ist sie aus hygienischen Gründen auch nicht unbedenklich. Es ist insofern Verbesserungsbedarf angestrebt.

Die EP0738545A1 offenbart eine Vorrichtung zum Reinigen von Absauggefäßen oder Sekretbehälter, welche einen Deckel mit einem Anschlussstutzen aufweisen. Dabei wird das Absauggefäß oder der Sekretbehälter verschlossen in die Vorrichtung eingeführt und über den Anschlussstutzen mit einem unter Druck stehenden Medium beaufschlagt, wodurch der Deckel abgesprengt wird.

Es ist die Aufgabe der Erfindung, eine Reinigungseinrichtung gemäß dem Oberbegriff von Anspruch 1 dahingehend zu verbessern, dass eine vereinfachte und gleichzeitig hygienischere Handhabung für ein mit einem Deckel versehenes Steckbecken ermöglicht ist.

Zur Lösung dieser Aufgabe wird mit der Erfindung vorgeschlagen eine Reinigungseinrichtung der vorstehend genannten Art, die sich durch eine Öffnungsvorrichtung für einen Deckel eines Steckbeckens auszeichnet.

Die erfindungsgemäße Reinigungseinrichtung ist im Unterschied zum Stand der Technik mit einer Öffnungsvorrichtung für einen Deckel eines Steckbeckens ausgerüstet. Diese Öffnungsvorrichtung ermöglicht ein automatisches Anheben des Steckbeckendeckels, sodass der Steckbeckeninhalt entfernt und das Steckbecken samt Deckel gereinigt werden kann. In vorteilhafterweise ist eine manuelle Entfernung des Deckels vom Steckbecken nicht mehr erforderlich. Das Steckbecken kann vielmehr mit aufgesetztem Deckel in die Steckbeckenaufnahme der Reinigungseinrichtung eingebracht werden. Bei einem bestimmungsgemäßen Verschwenken der Tür der Reinigungseinrichtung schwenkt der Deckel des Steckbeckens automatisch bei Seite, womit das Steckbecken als solches freigegeben wird und der darin befindliche Inhalt ausströmen kann. Während des nachfolgenden Reinigungsverfahrens erfolgt eine ordnungsgemäße Reinigung sowohl des Deckels, als auch des Steckbeckengrundkörpers, dass heißt des gesamten Steckbeckens. Nach erfolgreich durchgeführter Reinigung ist die Reinigungseinrichtung durch verschwenken der Tür in die Öffnungsstellung zu öffnen. Infolge dieser Verschwenkbewegung schwenkt der Deckel zurück in seine das Steckbecken abdeckende Stellung, sodass ein Bediener mit nur einem Handgriff das Steckbecken nebst darauf angeordnetem Deckel der Steckbeckenaufnahme der Reinigungseinrichtung entnehmen kann.

Mit der erfindungsgemäßen Reinigungseinrichtung wird insgesamt eine Steckbeckenreinigungsanlage vorgeschlagen, die es ermöglicht, Steckbecken samt Deckel zu reinigen, wobei der Deckel vor Beginn der Reinigung nicht manuell vom Steckbecken zu entfernen und in eine dafür vorgesehene Halterung einzusetzen ist. Das Steckbecken kann vielmehr nebst Deckel in die Steckbeckenaufnahme eingeführt und alsdann der Reinigungsvorgang in gewohnter Weise durchgeführt werden. Die erfindungsgemäße Reinigungseinrichtung erlaubt somit bei gleichzeitiger Verbesserung der Hygienebedingungen eine vereinfachte Handhabe.

Gemäß einem weiteren Merkmal der Erfindung ist vorgesehen, dass die Steckbeckenaufnahme über einen daran verschwenkbar angeordneten Tragarm verfügt. Dieser Tragarm trägt seinerseits verschwenkbar einen Greifer. Zusammen bilden der Tragarm und der Greifer einen Teil der Öffnungsvorrichtung für einen Deckel eines Steckbeckens.

Der Greifer dient in bestimmungsgemäßen Verwendungsfall der Aufnahme einer Deckelhandhabe. Diese kann als Knauf, Knopf, Bügel und dergleichen ausgebildet sein.

Der Greifer ist in seiner relativen Lageausrichtung zur übrigen Steckbeckenaufnahme derart positioniert, dass bei einem Einführen eines mit einem Deckel versehenen Steckbeckens in die Steckbeckenaufnahme die Deckelhandhabe vom Greifer erfasst wird. Bei einem anschließenden Verschwenken der den Reinigungsraum verschließenden Tür fällt der Deckel der Schwerkraft folgend vom Grundkörper des Steckbeckens herunter, wobei er vom Greifer bzw. vom Tragarm aufgenommen in eine durch die Geometrie des Tragarms vorgegebene Position verschwenkt wird. In dieser Position ist der vom Grundkörper des Steckbeckens bereitgestellte Volumenraum freizugänglich, sodass sich etwaige darin befindliche Körperflüssigkeiten oder dergleichen ausströmen können. Auch eine Reinigung des Steckbeckens ist in dieser Position des Deckels möglich.

Die Steckbeckenaufnahme verfügt gemäß einem weiteren Merkmal der Erfindung über eine Stützeinrichtung. Diese Stützeinrichtung dient dazu, den Deckel des Steckbeckens in verschwenktem Zustand abzustützen, dass heißt ihn in seiner relativen Lage zu sichern, sodass eine bestimmungsgemäße Reinigung durchgeführt werden kann.

Die Stützeinrichtung ist gemäß einem weiteren Merkmal der Erfindung bügelförmig ausgebildet und verfügt bevorzugter Weise über einen einstückig damit ausgebildeten Stützfortsatz, der in Richtung auf die Steckbeckenaufnahme ausgerichtet ist. Dieser Fortsatz dient der zusätzlichen Stabilisierung des Deckels im verschwenkten Zustand. Er dient als zusätzliche Abstützhilfe für den Deckel.

Gemäß einem weiteren Merkmal der Erfindung ist ein vom Greifer aufnehmbarer Adapter vorgesehen. Dieser Adapter kann als "Griffadapter" bezeichnet werden. Er dient dazu, in Ihrer geometrischen Ausgestaltung unter Umständen unterschiedliche Deckelgriffe mit ein und demselben Greifer verwendbar auszugestalten. Der Adapter fungiert insofern als Zwischenstück zwischen Greifer und Deckelgriff und ermöglicht die Kompatibilität von Greifer und Deckelgriff. Dabei ist jeder Deckelgriffgeometrie ein bestimmter Adapter zugeordnet.

Es ist bevorzugter Weise vorgesehen, dass der Greifer in seiner geometrischen Ausgestaltung auf eine bestimmte Deckelgriffausgestaltung abgestellt ist. Es kann beispielsweise vorgesehen sein, dass der Greifer nach Art einer Gabel ausgebildet ist, um einen als Deckelknauf ausgebildeten Griff hintergreifen zu können. Kommen nun Steckbecken mit Deckeln zum Einsatz, die über eine andere Griffausgestaltung verfügen, so ist gegebenfalls eine Verwendung in Kombination mit einem standardmäßig ausgebildeten Greifer nicht möglich. Um hier die Kompatibilität, dass heißt die Verwendungsmöglichkeit auch mit derlei Griffausgestaltungen sicherstellen zu können, wird die Verwendung eines Adapters vorgeschlagen. Dieser wird als Zwischenstück am Greifer befestigt und ermöglicht in Entsprechung seiner geometrischen Ausgestaltung die Aufnahme entsprechend ausgebildeter Deckelgriffe.

Weitere Merkmale und Vorteile der Erfindung ergeben sich aus der nachfolgenden Beschreibung anhand der Figuren.
- Figur 1:: in schematisch perspektivischer Ansicht eine Reinigungseinrichtung nach der Erfindung;
- Figur 2:: in schematisch perspektivischer Ansicht eine Reinigungsvorrichtung nach der Erfindung mit geöffneter Tür;
- Figur 3:: in teilgeschnittener Seitenansich in schematischer Darstellung eine Reinigungseinrichtung nach der Erfindung;
- Figur 4:: in schematischer Darstellung eine Tür der erfindungsgemäßen Reinigungseinrichtung in geöffneter Stellung und
- Figur 5:: die Tür nach Figur 4 in geschlossener Stellung

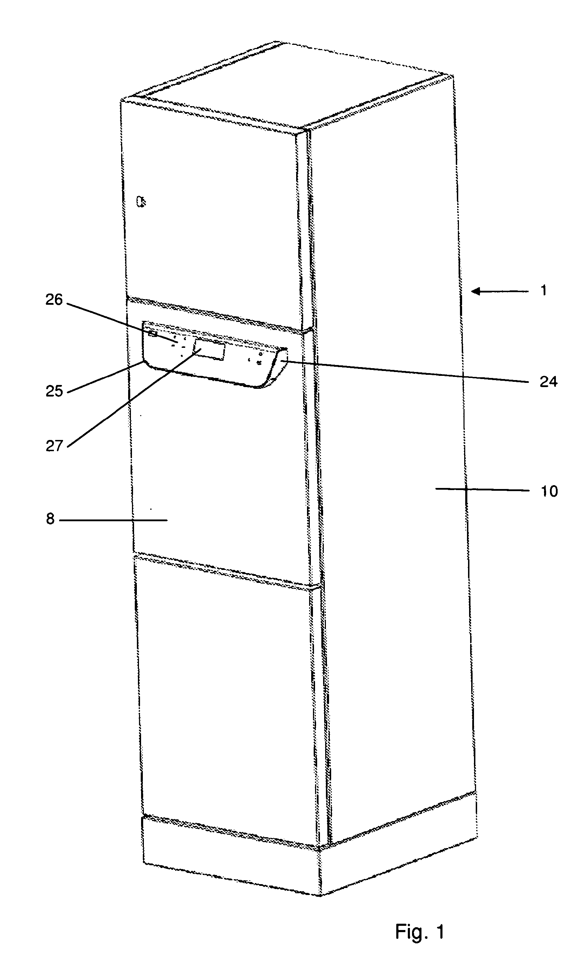

Figur 1 lässt in schematisch perspektivischer Ansicht eine Reinigungseinrichtung 1 erkennen. Diese dient der Reinigung von insbesondere Steckbecken 2, Urinflaschen 4 und/oder dergleichen und kann insofern auch als Steckbeckenreinigungseinrichtung bezeichnet werden.

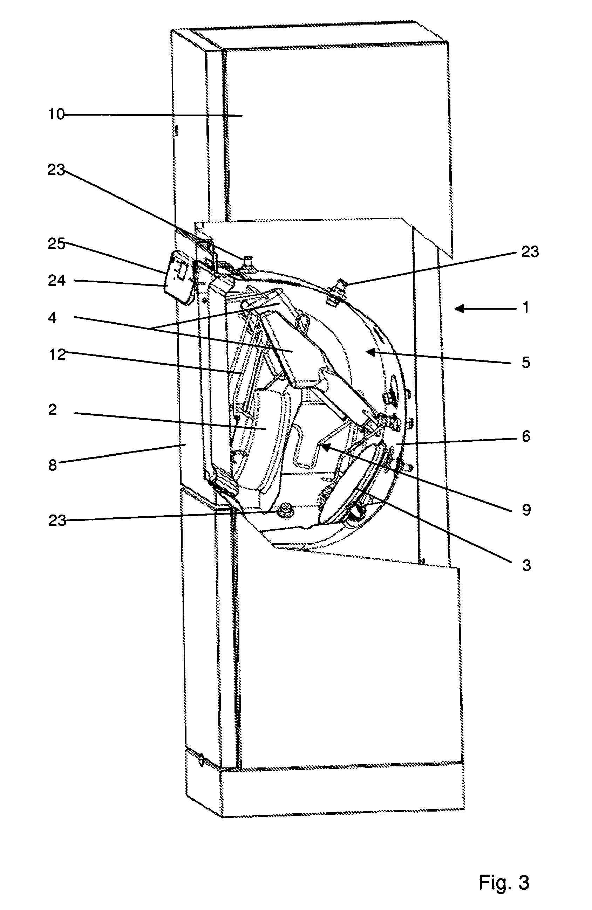

Die Reinigungseinrichtung 1 weist in an sich bekannter Weise ein Gehäuse 10 auf. Dieses nimmt - wie insbesondere die teilgeschnittene Seitenansicht nach Figur 3 erkennen lässt - einen Einsatz 6 auf. Dieser Einsatz 6 definiert den eigentlichen Reinigungsraum 5.

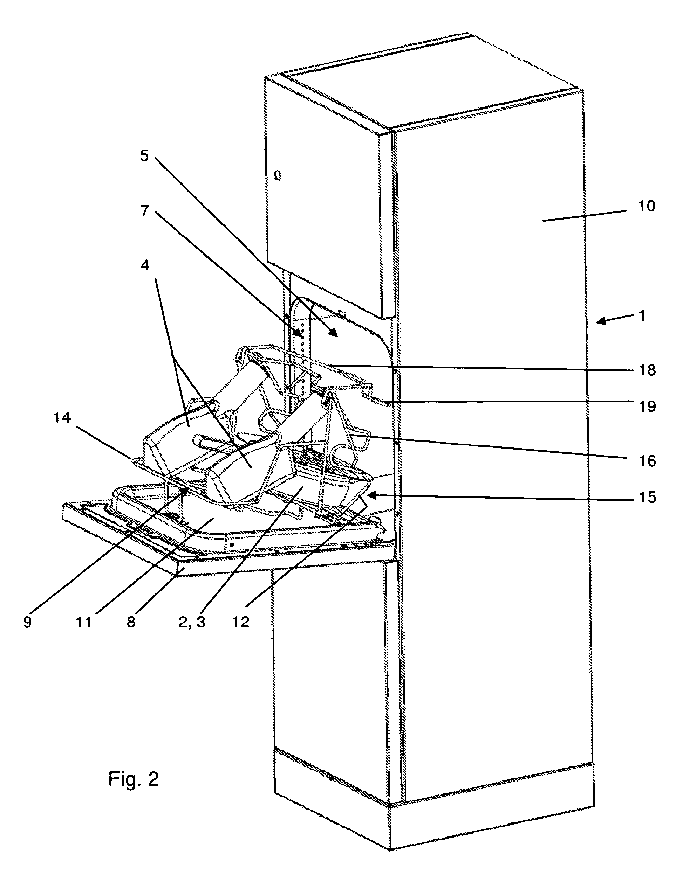

Der Reinigungsraum 5 ist über eine Beschickungsöffnung 7 zugänglich. Dies lässt insbesondere die Darstellung nach Figur 2 erkennen. Die Beschickungsöffnung 7 ist mittels einer Tür 8 verschließbar. Der geöffnete Zustand ist in Figur 2 dargestellt. Die Figuren 1 und 3 zeigen jeweils den geschlossenen Zustand.

Außenseitig der Tür 8 ist eine Bedieneinrichtung 25 angeordnet. Diese stellt einen Griff 24 bereit, der von einem Verwender zum Öffnen und/oder Schließen der Tür 8 ergriffen werden kann. Die Bedieneinrichtung 25 stellt eine Mehrzahl von Bedienelementen 26 sowie ein Display 27 bereit. Über die Bedienelemente 26 kann die Reinigungseinrichtung 1 bedient werden, zum Beispiel über die Auswahl vorgegebener Reinigungsprogramme. Das Display 27 dient insbesondere der Anzeige von ausgewählten Spül- oder Reinigungsprogrammen, noch verbleibender Reinigungszeit und/oder dergleichen.

Reinigungsraumseitig verfügt die Tür 8 über eine Steckbeckenaufnahme 9. Diese ist nach Art eines Drahtgestells ausgebildet und ermöglicht an den dafür vorgesehenen Plätzen die Aufnahme von Steckbecken 2, Urinflaschen 4 und/oder dergleichen, wie insbesondere eine Zusammenschau der Darstellungen nach den Figuren 2 und 3 zeigt.

Die Steckbeckenaufnahme 9 ist auf der Türinnenseite 11 der Tür 8 angeordnet. Bei einem Verschwenken der Tür 8 verschwenkt die Steckbeckenaufnahme 9 mit. Eine Beladung der Reinigungseinrichtung 1 findet bei geöffneter Tür 8 statt. Die zu reinigenden Steckbecken 2 und/oder Urinflaschen 4 werden in die Steckbeckenaufnahme 9 benutzerseitig eingebracht. Dies zeigt Figur 2. Nach einer Bestückung der Steckbeckenaufnahme 9 ist die Tür 8 in die Verschlussstellung gemäß Figur 3 zu verschwenken. Infolge dieser Verschwenkbewegung schwenken die zu reinigenden Steckbecken 2 und/oder Urinflaschen 4 in den vom Einsatz 6 bereitgestellten Reinigungsraum 5 ein, wie dies Figur 3 erkennen lässt.

In dieser Stellung der Steckbecken 2 und/oder Urinflaschen 4 kann eine Reinigung derselben durchgeführt werden. Zu diesem Zweck verfügt der Einsatz 6 über Düsen 23, über die Wasser und gegebenfalls auch Chemikalien in den Reinigungsraum 5 eingebracht werden können.

Die Steckbeckenaufnahme 9 stellt erfindungsgemäß eine Öffnungsvorrichtung 28 für den Deckel 3 eines Steckbeckens 2 zur Verfügung. Mittels dieser Öffnungsvorrichtung 28 findet ein gezieltes Verschwenken eines Deckels 3 eines Steckbeckens 2 statt, was es ermöglicht, das Steckbecken 2 mit darauf befindlichem Deckel 3 in die Steckbeckenaufnahme 9 einzubringen, wobei es im Unterschied zum Stand der Technik nicht erforderlich ist, den Deckel 3 zuvor manuell vom Grundkörper des Steckbeckens 2 zu entfernen. Bei einem Verschwenken der Tür 8 aus der Öffnungsstelllung in die geschlossene Stellung sorgt die Öffnungsvorrichtung 28 vielmehr für eine automatische Verschwenkung des Deckels 3, wodurch das Steckbecken 2 geöffnet und der vom Steckbecken 2 bereit gestellte Volumenraum zur Aufnahme von Körperflüssigkeiten und/oder dergleichen zugänglich wird.

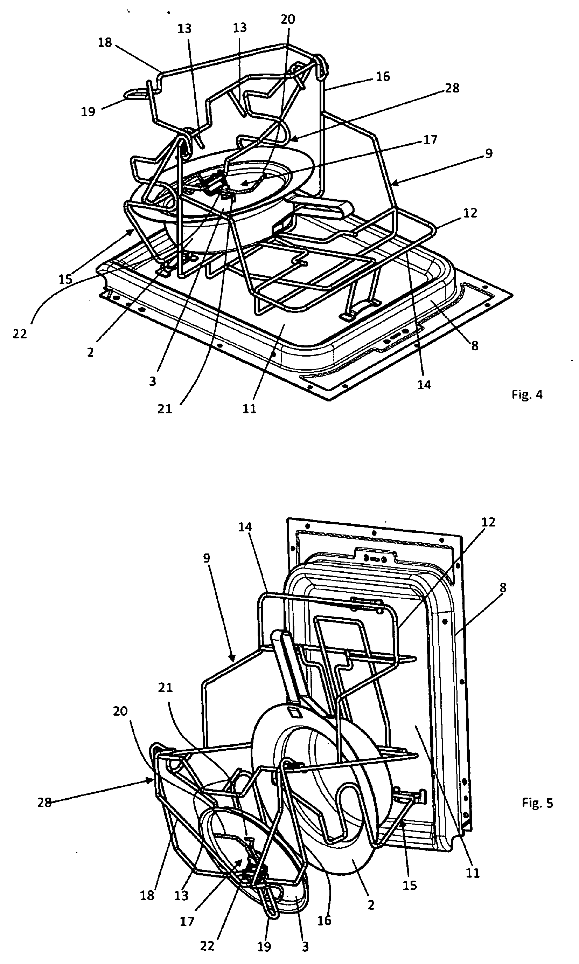

Die erfindungsgemäße Öffnungsvorrichtung 28 verfügt über einen Tragarm 16. Der Tragarm 16 ist verschwenkbar am Gestell 12 der Steckbeckenaufnahme 9 angeordnet. Das Gestell 12 dient dabei nicht nur der Aufnahme von Steckbecken 2. Es können, wie die Figuren 2 und 3 zeigen, auch Urinflaschen 4 aufgenommen werden, zu welchem Zweck das Gestell 12 mit Bezug auf die Zeichnungsebene nach Figur 5 oberseitig eine Reling 14 aufweist.

Der vom Gestell 12 bereit gestellte Bereich 15 dient der Aufnahme eines Steckbeckens 2. Dieser Sachzusammenhang ergibt sich insbesondere aus den Figuren 4 und 5. Ein zu reinigendes Steckbecken wird samt Deckel 3 in den vom Gestell 12 bereit gestellten Bereich 15 eingesetzt. Dies geschieht bei geöffneter Tür 8, wie sich insbesondere aus Figur 4 ergibt. Der verschwenkbar am Gestell 12 angeordnete Tragarm 16 befindet sich in dieser Position der Tür 8 in seiner herunter geschwenkten Stellung, wie die Darstellung nach Figur 4 erkennen lässt. Der Tragarm 16 stellt einen Greifer 17 bereit, der im gezeigten Ausführungsbeispiel nach Art einer Gabel ausgebildet ist. Beim Einsetzen des Steckbeckens 2 in den Bereich 15 hintegreift der Greifer 17 den vom Deckel 3 zwecks Handhabung bereitgestellten Knauf 22. Die Öffnungsvorrichtung 28 verfügt darüber hinaus über eine Stützeinrichtung 18. Diese ist bügelförmig ausgebildet und gleichfalls am Gestell 12 angeordnet. Die Stützeinrichtung 18 dient der Abstützung des Deckels 3, und zwar in der verschwenkten Stellung, dass heißt während des Reinigungsvorgangs. Dieser Sachzusammenhang ist insbesondere der Darstellung nach Figur 5 zu entnehmen.

Die Stützeinrichtung 18 verfügt zwecks Abstützung des Deckels 3 bevorzugterweise über einen einstückig damit ausgebildeten Fortsatz 19. Dieser Fortsatz 19 ist bügelartig ausgebildet und dient der lagesicheren Abstützung des Deckels 3 relativ gegenüber dem Gestell 12.

Wie ebenfalls die Figur 5 am deutlichsten erkennen lässt, verfügt der Greifer 17 über zwei Arme 20 und 21. Diese sind nach Art einer Gabel relativ zueinander ausgebildet, wobei sie öffnungsseitig aufgebogen sind, was ein Einführen des Knaufs 22 des Deckels 3 in die vom Greifer 17 bereit gestellte Öffnung erleichtert.

Das Gestell 12 stellt ferner Dorne 13 zur Verfügung. Diese dienen in Kombination mit der schon vorerläuterten Reling 14 der Abstützung von insbesondere Urinflaschen 4.

Die Funktionsweise der erfindungsgemäßen Öffnungsvorrichtung 28 ist die folgende:
Beim Beladen der Reinigungseinrichtung 1 - auch Steckbeckenspülautomat genannt - wird das Steckbecken 2 mit aufliegendem Deckel 3 in den Bereich 15 zur Aufnahme geschoben. Dabei wird der als Deckelgriff dienende Knauf 22 in die vom Greifer 17 bereitgestellte Öffnung eingeführt. Der Greifer 17 kann insofern auch als Deckelöffner bezeichnet werden.

Der Greifer 17 ist am Tragarm 16 angeordnet, der wiederum verschwenkbar am Gestell 12 der Steckbeckenaufnahme 9 angelenkt ist.

Beim Schließen der Tür 8 schwenkt der Tragarm 16 zusammen mit dem Greifer 17 weg und hebt dabei den Deckel 3 vom Grundkörper des Steckbeckens 2 ab. Der Tragarm 16 und der Deckel 3 werden durch die aus Stützeinrichtung 18 und Fortsatz 19 gebildete Deckelstütze in einer definierten Position gefangen. Anschließend gleitet der Greifer 17 inklusive dem davon gehaltenen Deckel 3 an dem auch als Nase zu bezeichnenden Fortsatz 19 der Stützeinrichtung 18 nach unten. Dabei kippt der Greifer 17 mit dem Deckel 3 über und bleibt in der durch die geometrische Ausgestaltung der Steckbeckenaufnahme 9 festgelegten Position liegen. In dieser Position hängt der Deckel 3 am Greifer 17 und stützt sich dabei am oberen Teil der Stützeinrichtung 18 ab.

In dieser in Figur 5 gezeigten Position kann eine ordnungsgemäße Reinigung des Steckbeckens 2 stattfinden.

Wird nun die Tür 8 nach erfolgreich durchgeführtem Reinigungsvorgang wieder geöffnet, so schwenkt der Tragarm 16 zusammen mit dem daran angeordneten Deckel 3 zurück, wobei sich der Deckel 3 auf den Grundkörper des Steckenbeckens 2 auflegt. Das Steckbecken 2 kann nun samt Deckel 3 der Reinigungseinrichtung 1 wieder entnommen werden.

## Patentansprüche

1. Reinigungseinrichtung (1) für einen Grundkörper und einen Deckel (3) umfassende Steckbecken (2), mit einer Tür (8) und mit einem Gehäuse (10), das einen Reinigungsraum (5) bereitstellt, wobei der Reinigungsraum (5) eine Beschickungsöffnung (7) aufweist, die mittels der Tür (8) fluiddicht verschließbar ist, welche Tür (8) reinigungsraumseitig eine Steckbeckenaufnahme (9) trägt,
**gekennzeichnet durch** eine Öffnungsvorrichtung (28) für einen Deckel (3) eines Steckbeckens (2), welche einen verschwenkbar an der Steckbeckenaufnahme (9) angeordneten Tragarm (16) mit einem verschwenkbar an dem Tragarm (16) angeordneten Greifer (17), der zur Aufnahme einer Deckelhandhabe dient, aufweist, derart dass bei einem Verschwenken der den Reinigungsraum (5) verschließenden Tür (8) der Deckel (3) oder Schwerkrafgt folgend vom Grundkörper des Steckbeckens (2) herunterfällt, wobei er vom Greifer (17) aufgenommen und in eine **durch** die Geometrie des Tragarms (16) vorgegebene Position verschwenkt wird.

2. Reinigungseinrichtung (1) nach Anspruch 1,
**gekennzeichnet durch** eine an der Steckbeckenaufnahme (9) angeordnete Stützeinrichtung (18).

3. Reinigungseinrichtung (1) nach einem der vorhergehenden Ansprüche,
**dadurch gekennzeichnet,**
**dass** der Greifer (17) nach Art einer Gabel ausgebildet ist und öffnungsseitig eine trichterförmige Erweiterung aufweist.

4. Reinigungseinrichtung (1) nach einem der Ansprüche 2 oder 3,
**dadurch gekennzeichnet,**
**dass** die Stützeinrichtung (18) bügelförmig ausgebildet ist und einen damit vorzugsweise einstückig ausgebildeten Stützfortsatz (19) aufweist, der in Richtung auf die Steckbeckenaufnahme (9) ausgerichtet ist.

5. Reinigungseinrichtung (1) nach einem der vorhergehenden Ansprüche,
**dadurch gekennzeichnet,**
**dass** der Tragarm (16) bügelförmig ausgebildet ist.

6. Reinigungseinrichtung (1) nach einem der vorhergehenden Ansprüche,
**gekennzeichnet durch** einen vom Greifer 17 aufnehmbaren Adapter.

7. Reinigungseinrichtung (1) nach einem der vorhergehenden Ansprüche,
**dadurch gekennzeichnet,**
**dass** der Tragarm (16), der Greifer (17) und die Stützeinrichtung (18) jeweils aus gebogenem Draht gebildet sind.

## Claims

1. Cleaning device (1) for bedpans (2) comprising a main body and a lid (3), comprising a door (8) and a housing (10) which provides a cleaning space (5), the cleaning space (5) having a load opening (7) which can be sealed in a fluid-tight manner by means of the door (8), which door (8) carries a bedpan-receiving fixture (9) on the cleaning space side, **characterised by** an opening device (28) for a lid (3) of a bedpan (2), which opening device has a carry arm (16) which is pivotally arranged on the bedpan-receiving fixture (9) and which has a gripper (17) which is pivotally arranged on the carry arm (16), which gripper is used to receive a lid handle, such that when the door (8) sealing the cleaning space (5) pivots, the lid (3) falls away from the main body of the bedpan (2) as a result of gravity, said lid being received by the gripper (17) and pivoted into a position which is determined by the geometry of the carry arm (16).

2. Cleaning device (1) according to claim 1, **characterised by** a support device (18) arranged on the bedpan-receiving fixture (9).

3. Cleaning device (1) according to any of the preceding claims, **characterised in that** the gripper (17) is configured in the manner of a fork and has a funnel-shaped extension on the opening side.

4. Cleaning device (1) according to either claim 2 or claim 3, **characterised in that** the support device (18) has a stirrup-shaped configuration and a support projection (19) which is preferably formed in one piece therewith and which faces the bedpan-receiving fixture (9).

5. Cleaning device (1) according to any of the preceding claims, **characterised in that** the carry arm (16) has a stirrup-shaped configuration.

6. Cleaning device (1) according to any of the preceding claims, **characterised by** an adapter which can be received by the gripper 17.

7. Cleaning device (1) according to any of the preceding claims, **characterised in that** the carry arm (16), the gripper (17) and the support device (18) are each formed from bent wire.

## Revendications

1. Equipement de nettoyage (1) pour des bassins de lit (2) comprenant un corps principal et un couvercle (3), avec une porte (8) et un carter (10) qui réalise un espace de nettoyage (5), l'espace de nettoyage (5) présentant une ouverture de chargement (7) qui peut être fermée de façon étanche aux fluides au moyen de la porte (8), laquelle porte (8) porte, côté espace de nettoyage, un logement pour bassins de lit (9),
**caractérisé par** un dispositif d'ouverture (28) destiné à un couvercle (3) d'un bassin de lit (2) et qui présente un bras porteur (16) disposé de façon pivotante sur le logement pour bassins de lit (9) et doté d'un grappin (17) qui est disposé de façon pivotante sur le bras porteur (16) et qui sert à loger une prise de couvercle de sorte que, lors d'un pivotement de la porte (8) fermant l'espace de nettoyage (5), le couvercle (3), du fait de la gravité, tombe du corps principal du bassin de lit (2), et est alors reçu par le grappin (17) et est conduit à pivoter dans une position prédéfinie par la géométrie du bras porteur (16).

2. Equipement de nettoyage (1) selon la revendication 1,
**caractérisé par** un équipement de support (18) disposé sur le logement pour bassins de lit (9).

3. Equipement de nettoyage (1) selon une des revendications précédentes,
**caractérisé en ce que**
le grappin (17) est constitué à la façon d'une fourche et présente, côté ouverture, un élargissement en forme d'entonnoir.

4. Equipement de nettoyage (1) selon une des revendications 2 ou 3,
**caractérisé en ce que**
l'équipement de support (18) est constitué en forme d'étrier et présente un prolongement de support (19) qui est constitué de préférence d'un seul tenant avec lui et qui est orienté dans la direction du logement pour bassins de lit (9).

5. Equipement de nettoyage (1) selon une des revendications précédentes,
**caractérisé en ce que**
le bras porteur (16) est constitué en forme d'étrier.

6. Equipement de nettoyage (1) selon une des revendications précédentes,
**caractérisé par** un adaptateur pouvant être logé par le grappin 17.

7. Equipement de nettoyage (1) selon une des revendications précédentes,
**caractérisé en ce que**
le bras porteur (16), le grappin (17) et l'équipement de support (18) sont formés respectivement de fil métallique courbé.
